# EUROPEAN PATENT SPECIFICATION

(11) **EP 3 527 123 B1**
(45) Date of publication and mention of the grant of the patent: **31.08.2022**
(21) Application number: 18156906.2
(22) Date of filing: 15.02.2018
(51) Int. Cl.: A61B 5/00

(54) **IMAGE PROCESSING METHOD AND APPARATUS USING ELASTIC MAPPING OF VASCULAR PLEXUS STRUCTURES**
BILDVERARBEITUNGSVERFAHREN UND -VORRICHTUNG MIT ELASTISCHER ABBILDUNG VON GEFÄSSPLEXUSSTRUKTUREN
PROCÉDÉ ET APPAREIL DE TRAITEMENT D'IMAGE UTILISANT UN MAPPAGE ÉLASTIQUE DE STRUCTURES DE PLEXUS VASCULAIRE

(43) Date of publication of application: 21.08.2019
(73) Proprietor: Leica Instruments (Singapore) Pte. Ltd., 608924 Singapore (SG)
(72) Inventor: THEMELIS, George, 88131 Lindau (DE)
(74) Representative: Grünecker Patent- und Rechtsanwälte PartG mbB

(56) References cited:
- WO-A1-2010/046838
- WO-A1-2015/023990
- WO-A1-2015/084143
- THOMAS WINDHEUSER ET AL: "Geometrically consistent elastic matching of 3D shapes: A linear programming solution", COMPUTER VISION (ICCV), 2011 IEEE INTERNATIONAL CONFERENCE ON, IEEE, 6 November 2011 (2011-11-06), pages 2134-2141, XP032101442, DOI: 10.1109/ICCV.2011.6126489 ISBN: 978-1-4577-1101-5

## Description

The invention relates to an image processing method and a medical observation device for displaying soft tissue images, in particular in real time during surgery.

Image-guided surgery is nowadays commonly used for certain kind of surgical operations, such as brain surgery. Image-guided surgery uses stored pre-operative three-dimensional information in the form of image data about the operation area. Such pre-operative three-dimensional image data may, for example, have been obtained using magnetic resonance imaging. During surgery, the pre-operative information is visually aligned with the actual optical view of the tissue to be operated on. Using the pre-operative three-dimensional information, tissue structures, such as tumors or vascular plexus structures, may be visualized that are otherwise invisible under the visible tissue surface.

Use of the pre-operative three-dimensional information helps the surgeon to find and reach a certain area of the tissue, to avoid sensitive tissue such as nerves, arteries and veins, and/or to remove certain tissue effectively, such as a tumor.

A method and apparatus for quantitative and depth resolved fluorescence and reflective imaging for surgical guidance is known from WO 2015/023990 A1. A patient's brain is analyzed by a diagnostic tool such as MRI, PMR or CT in order to provide a three-dimensional model of the tissue. A surgeon may identify structures in the tissue and mark them during pre-operative planning. During surgery, fluorophore concentrations in the tissue are imaged using depth resolved fluorescence microscopy.

For the alignment of the pre-operative three-dimensional information to the actual view of the surgeon, either stereo infrared cameras or sensors for detecting optical or electromagnetic markers fixed on the patient's body are typically used.

The spatial accuracy of these methods is not sufficient, however, if surgery is performed on soft tissue, which can shift and deform relative to the markers.

Therefore, in existing systems, at one point during surgery, the surgeon needs to align the pre-operative three-dimensional information manually with his actual view. This consumes time and time is critical for all surgery. Moreover, the surgeon is distracted from the surgery, which is also to be avoided.

It is therefore the object of the invention to provide a method and device which allow the performance of image-guided surgery even if the surgery is performed on soft tissue which may easily deform and shift during the operation.

According to the inventive method, this object is solved by an image processing method for displaying soft tissue images as defined in claim 1.

To solve the above problem, the medical observation device for the observation of soft tissue images is configured as defined in claim 6.

The method and device according to the invention allow the continuous performance of image-guided surgery in soft tissue even if the tissue deforms and moves within the body without the need to manually realign the pre-operative image data to the interoperative image data. This is because the structure which is used for elastically matching the pre-operative three-dimensional image data is a structure which is part of the soft tissue and thus deforms and moves together with the soft tissue. The pre-operative three-dimensional image data are thus mapped continuously to what the surgeon actually sees. In fact, the visual information that is available to the surgeon is itself used to align the pre-operative three-dimensional image data.

The image processing method and the medical observation device according to the invention may be improved by adding one or more of the following features. Each of the following features can be added independently of the remaining features. Each of the following features has its own advantageous technical effect. Further, the following features can all be added equally to both the method and the device.

The modules described above can be implemented in software, hardware or a combination of both software and hardware. Further, the differences between the particular modules are primarily functional. Different modules may thus be comprised of a single or a plurality of electric components and/or a single logical unit, such as a single sub-routine.

The output image data may be displayed on a display assembly, which may be part of the medical observation device.

The step of elastic matching may include a technique as is described in Gee, J. C.; Reivich, M.; and Bajcsy, R.: "Elastically Deforming a Three-Dimensional Atlas to Match Anatomical Brain Images" (1993). IRCS Technical Reports Series. 192. The step of identifying a vascular plexus structure may use the method as described in Suri, J.S.; Laxminarayan, S. [eds]: "Angiography and Plaque Imaging: Advanced Segmentation Techniques", CRC Press, 2003, pp. 501-518, and Rouchdy, Y.; Cohen, L.: "A Geodesic Voting Method of Tubular Tree and Centrelines", DOI: 10.1109/ISBI.2011.5872566, 2011, pp. 979-983. The matching module may in particular be configured to execute a matching routine described in any of these references.

In one embodiment, the identification of the at least one vascular plexus structure may be done preferably exclusively with interoperative fluorescent-light image data. Such fluorescent-light image data may be obtained by injecting a fluorophore, such as indocyanine green, into the tissue. The camera assembly may comprise an optical filter assembly, such as a band-pass filter assembly, of which the pass band is restricted to the fluorescence spectrum of the fluorophore. As the fluorophore is transported by the blood, the vascular plexus structure may be more easily defined in the fluorescent-light image. The medical observation device may have an illumination assembly, having an illumination spectrum which comprises light in wavelengths that trigger fluorescence of the fluorophore. The illumination spectrum may be restricted to these fluorescence-triggering wavelengths.

The interoperative image data may, in another embodiment, contain both white-light image data and fluorescent-light image data. The white-light image data may be used to present output image data to the surgeon that represent what he would see with his own eyes. For this, the illumination spectrum may also comprise wavelengths of the visible spectrum, in particular white light.

The medical observation device may be one of a microscope and an endoscope.

The interoperative image data may be two-dimensional, three-dimensional or multidimensional data. Three-dimensional interoperative image data may, for example, be acquired by a microscope using z-stacking or a stereoscopic setup, or a SCAPE or SPIM microscope.

The interoperative image data may be recorded simultaneously in more than three different wave bands using, for example, more than one camera, a multi-spectral camera and/or a hyper-spectral camera.

The elastic mapping may include or consist of the step of elastically matching the at least one identified vascular plexus structure in the pre-operative three-dimensional image data to the corresponding at least one identified vascular plexus structure in the interoperative image data. The mapping used for the at least one vascular plexus structure may be used for the rest of the pre-operative three-dimensional image data.

The accuracy and reliability of the mapping of the pre-operative three-dimensional image data to the interoperative image data depends on how accurately the at least one vascular plexus structure may be recognised. As already stated above, pattern recognition may be performed on the fluorescent-light interoperative image data, using regions in which the fluorophore is located and which therefore have high fluorescent-light intensity.

The method in another embodiment may comprise the step of identifying at least one arterial vascular plexus structure within the interoperative image data, and/or at least one venous vascular plexus structure within the interoperative image data. For identifying the type of vascular plexus structure and for generating output image data in which the different types of vascular plexus structures are marked in different false colors, the system and method described in European patent application EP 17 174 047.5 may be used (published as EP 3 410 394 A1 and prior art under Article 54(3) EPC).

Additionally or alternatively, the at least one vascular plexus structure may be identified using interoperative image data which have been acquired in at least one of a plurality of separate wavelengths e.g. by a multispectral and/or hyperspectral camera. The multi- and/or hyperspectral interoperative image data may be unmixed and/or processed to show the distribution of at least one of arterial or venous blood, or the respective blood vessels. A method and apparatus for identifying the at least one vascular plexus structure is described e.g. in Matthew B. Bouchard, Brenda R. Chen, Sean A. Burgess, and Elizabeth M. C. Hillman, "Ultra-fast multispectral optical imaging of cortical oxygenation, blood flow, and intracellular calcium dynamics," Opt. Express 17, 15670-15678 (2009).

Another measure for facilitating the identification of the at least one vascular plexus structure is to use at least one optical cross-polarizing filter assembly for reducing specular reflections. This method is described in European patent application EP 16 204 933.2, published as EP 3 336 597 A1 and prior art under Article 54(3) EPC.

A further step towards a more reliable identification of the at least one vascular plexus structure is to compute, at at least one location in the interoperative image data, the blood flow direction using the fluorescent-light image data. The blood flow direction can be computed at a given location by determining at least one of the temporal and derivative and the spatial derivative, as is described in European patent application EP 17 210 909.2, published as EP 3 505 059 A1 and prior art under Article 54(3) EPC. A location of the interoperative image data may respond to a single pixel or a coherent array of pixels. Additionally or alternatively, a principal component analysis may be used to determine blood flow direction as description in the above-mentioned European patent application EP 17 174 047.5.

The mapping of the pre-operative three-dimensional image data to the interoperative image data may be facilitated if in addition to the mere image data, additional information about the position of the interoperative image data, and thus the vascular plexus structure within the tissue, is available. For this, a position sensor may be provided for generating position data representative of the position of a field of view of the camera assembly. The position data may comprise at least one of focal length, field depth and distance setting of the camera assembly. The position data may comprise at least one of incremental position data and absolute position data. Incremental position data may be used to indicate the change of the position of the field of view from one frame of interoperative image data to a subsequent frame of interoperative image data. Absolute position data may be used for each frame of the interoperative image data to indicate absolute position with reference to a constant reference. Changes of the position data between subsequent frames of interoperative image data may then be obtained by computing differences of the respective absolute position data. The positioning data may, in one embodiment, be used in at least one of identifying the at least one vascular plexus structure in the three-dimensional data, elastically matching the pre-operative three-dimensional image data to the interoperative image data, and displaying the output image data.

Finally, the invention is also directed to a non-transitory computer-readable medium storing a programme causing a computer to execute the method in any of the above-described embodiments.

In the following, an embodiment of the invention is exemplarily described with reference to the accompanying drawing.

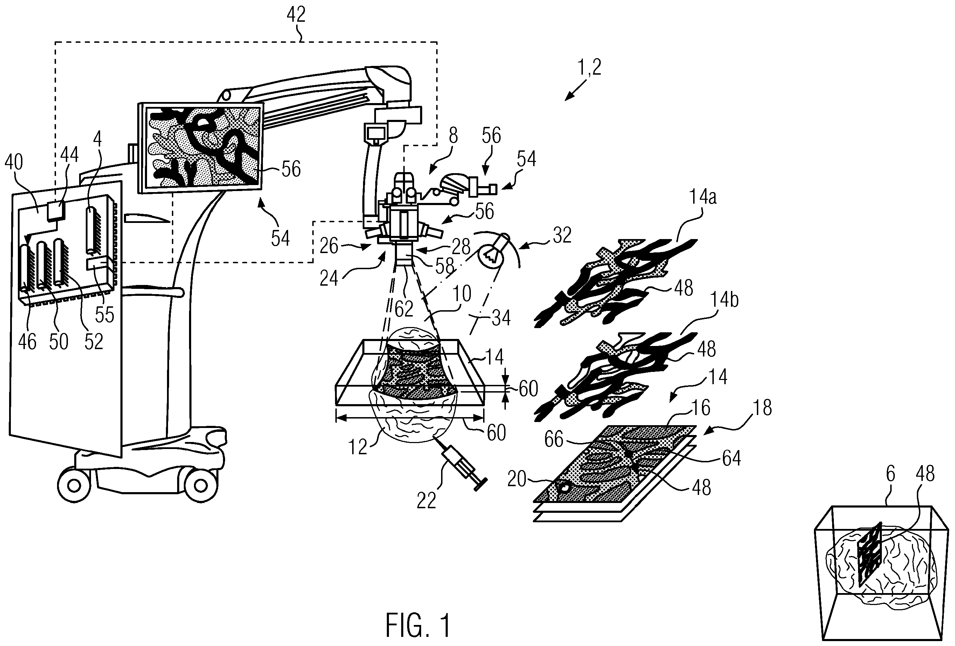

Fig. 1 shows an exemplary embodiment of the method and device according to the invention.

The configuration and function of an optical observation device 1 for observing live tissue, in particular during surgery, is explained. The medical observation device is shown to be a microscope 2 just for the purposes of explanation. The medical observation device 1 may also be an endoscope (not shown).

The medical observation device 1 comprises a memory assembly 4, in which pre-operative three-dimensional image data 6 are stored. The memory assembly 4 may comprise standard computer memory.

The medical observation device 1 further comprises a camera assembly 8, which has a field of view 10. During surgery, soft biological tissue 12, such as brain tissue, muscle tissue, lymph tissue or tissue of an internal organ or of other soft body parts, may be arranged in the field of view 10. During surgery, the camera assembly 8 acquires interoperative image data 14, which may be structured as a single input frame 16 or a time series 18 of input frames 16. The interoperative image data 14 may comprise or consist of pixels 20. The interoperative image data 14 may be two-dimensional, i.e. representing a plane in the field of view 10, three-dimensional, i.e. representing a volume in the field of view 10, or multidimensional image data which may e.g. comprises three-dimensional data in the field of view 10 at different spectral wavelengths.

The camera assembly 8 may comprise at least one of an RGB camera, a multi-spectral camera and a hyper-spectral camera.

The interoperative image data comprise or consist of fluorescent-light image data. Such fluorescent-light image data may be obtained when a fluorophore 22, such as indocyanine green, is injected into the tissue 12 and illuminated at wavelengths which trigger the fluorescence.

The camera assembly 8 may comprise one or more filter assemblies 24, which are only schematically shown in Fig. 1. The filter assembly 24 may comprise a filter arrangement 26 for blocking specular reflections. Examples of such a filter arrangement are described in the above-mentioned European patent application EP 16 204 933.2.

The filter assembly 24 may also comprise a band-pass filter arrangement 28 for restricting the light in the interoperative image data 14 to the fluorescence wavelengths of the at least one fluorophore 22. Such a filter arrangement is shown in European patent application EP 17 179 019.8, published as EP 3 422 072 A1 and prior art under Article 54(3) EPC.

The medical observation device 1 may further include an illumination assembly 32 for generating illumination light 34 having an illumination spectrum. The illumination spectrum may be restricted to or include wavelengths that trigger fluorescence of the at least one fluorophore 22. The illumination light 34 may further comprise or be restricted to wavelengths that match the reflectance spectrum of arterial blood. The illumination light 34 may be restricted to or comprise wavelengths that are matched to the reflectance spectrum of venous blood. Restricting the illumination spectrum of the illumination light 34 to a single or to preferably separate wavelengths reduced cross-talk between the various frequency bands. This facilitates an automatic analysis of the interoperative image data 14. Subsequent input frames 16 may have been acquired at different illumination spectra. Alternatively, the interoperative image data 14 contain information preferably in at least one of the visible-light spectra, e.g. in at least one of the reflective spectrum of arterial blood and venous blood, and the near-infrared spectrum, e.g. in the fluorescence wavelengths of the at least one fluorophore 22.

The medical observation device 1 further includes an image processor assembly 40, which only by way of example is shown as an integrated circuit in Fig. 1. The image processor 40 and its constituents may be software-implemented, hardware-implemented or be implemented as a combination of hardware and software. The memory assembly 4 may be part of the image processor 40. The image processor assembly 40 may comprise several modules which may be differentiated functionally and/or structurally. The image processor assembly 40 is connected to the camera assembly 8 by a data connection 42, which may be wired and/or wireless. An input interface 44 of the image processor assembly 40 may be adapted to acquire interoperative image data 14 from at least one camera assembly 8 and/or a storage where the interoperative image data 14 are stored or buffered, e.g. after pre-processing, such as memory assembly 4.

The image processor assembly 40 comprises a pattern-matching module 46 for identifying at least one vascular plexus structure 48 in the interoperative image data 14 and for identifying the at least one identified vascular plexus structure 48 in the pre-operative three-dimensional image data 6.

The at least one vascular plexus structure 48 may be identified e.g. in interoperative image data 14a which are restricted to the fluorescence spectrum of the fluorophore 22. In addition or alternatively, the at least one vascular plexus structure 48 may be identified in interoperative image data 14b which have been recorded in the visible-light spectrum and may in particular be restricted to at least one of the reflectance spectrum of arterial blood and venous blood. Algorithms for identifying a vascular plexus structure 48 in the interoperative image data 14 and then identifying this structure in the pre-operative three-dimensional image data 6 are, for example, given in Rouchdy, Y.; Cohen, L.: "A Geodesic Voting Method of Tubular Tree and Centrelines", DOI: 10.1109/ISBI.2011.5872566, pp. 979-983, and Suri, J.S.; Laxminarayan, S. (eds): "Angiography and Plaque Imaging: Advanced Segmentation Techniques", CRC Press, pp. 501-518. Further, a method of identifying vascular plexus structures by using a bolus of at least one fluorophore is described in the above-mentioned EP 17 174 047.5.

The image processor assembly 40 further comprises an elastic-matching module 50 for elastically matching the pre-operative three-dimensional image data 6 to the interoperative image data 14 based on the at least one identified vascular plexus structure 48. Again, an algorithm for performing such elastic matching is described in IRCS Technical Reports Series, 192, "Elastically Deforming a Three-Dimensional Atlas to Match Anatomical Brain Images" as given above. As a result of the elastic matching, the pre-operative three-dimensional image data 6 are shifted, rotated and/or distorted so that the identified vascular plexus structure 48 in both data coincides geometrically. For this, the step of elastic matching may also define a section through the pre-operative three-dimensional image data 6 which results in the field of view 10 represented in the interoperative image data 14. Further, if the camera assembly 8 comprises at least one of a multispectral camera and a hyperspectral camera for acquiring the interoperative image data 14, the blood vessel structure 48 and its type may be determined using the apparatus and method described in Opt. Express 17, 15670-15678, "Ultra-fast Multispectral Optical Imaging of Cortical Oxigenation, Blood Flow, and Intracellular Calcium Dynamics", as given above.

The image processor assembly 40 further comprises an image-forming module 52 for combining the elastically matched pre-operative three-dimensional image data 6 or a section thereof with the interoperative image data 14 into output image data 54. The image processor assembly may further comprise an output interface 55 for outputting the output image data 54. The output interface 55 may comprise at least one standard connector, such as an HDMI, DVI, RGB or any other suitable type of connector, and/or a wireless connection, including the matching data transmission protocol.

For displaying the output image data 54, the medical observation device 1 may comprise a display assembly 56, which may include stereoscopic display, for example an eyepiece of a microscope or endoscope and/or a monitor. The display assembly 56 may be connected to the output interface 55 by wire or wireless.

The computational burden for locating and/or identifying the identified vascular plexus structure 48 of the interoperative image data 14 in the pre-operative three-dimensional image data 6 may be high. This burden can be reduced if position information is provided as to where the field of view 10, the vascular plexus structure 48, and/or the interoperative image data 14 are located within the soft biological tissue 12. This information is useful if, for example, an elastic-matching process has already been successfully carried out for one input frame 16 of a time series 18 and then has to be repeated for subsequent input frame 16 of the time series 18 later during surgery. For this, the medical observation device 1 may comprise at least one position sensor 58 for acquiring and/or providing positioning data 60 representative of at least one of focal length, field depth and distance setting of an optical lens system 62 of the medical observation device 1 and size, distance from the optical lens system 62 and the image processor assembly 40, such as a dimension of the field of view 10.

The positioning data 60 may be input into at least one of the pattern-matching modules 46, the elastic-matching module 50 and the image-forming module 52 and be used in identifying the at least one identified vascular plexus structure 48 in the pre-operative three-dimensional image data 6, the elastic matching of the pre-operative three-dimensional image data 6 to the interoperative image data 14 and for forming the output image data 54 from the elastically matched pre-operative three-dimensional image data 6 and the interoperative image data 14.

The image processor assembly 40 may be adapted to compute the blood flow direction 64 at a location 66 in the at least one identified vascular plexus structure 48 as is described in the above-mentioned European patent application EP 17 210 909.2. A location may be a pixel 20 or a preferably coherent array of pixels in the interoperative image data 14. The tissue type of the identified vascular plexus structure 48, i.e. a differentiation between venous, arterial and capillary tissue and/or vessels may be carried out using the method and system described in the above-mentioned EP 17 174 047.5.

As described, the above-described system and method rely solely on information that is automatically acquired when the interoperative image data 14 are recorded. The system and method may further comprise positioning data 60 that result from the settings of the medical observation device 1 for acquiring the interoperative image data 14.

### REFERENCE NUMERALS

- 1: medical observation device
- 2: microscope
- 4: memory assembly
- 6: pre-operative three-dimensional image data
- 8: camera assembly
- 10: field of view
- 12: soft biological tissue
- 14: interoperative image data
- 14a: fluorescent-light interoperative image data
- 14b: visible-light interoperative image data
- 16: input frame
- 18: time series of input frames
- 20: pixel
- 22: fluorophore
- 24: filter assembly
- 26: filter arrangement for blocking specular reflections
- 28: band-pass filter arrangement for fluorescent light
- 32: illumination assembly
- 34: illumination light
- 40: image processor assembly
- 42: data connection
- 44: input interface
- 46: pattern-matching module
- 48: vascular plexus structure
- 50: elastic-matching module
- 52: image-forming module
- 54: output image data
- 55: output interface
- 56: display assembly
- 58: position sensor
- 60: positioning data
- 62: optical lens system
- 64: blood flow direction
- 66: location

## Claims

1. Image processing method for displaying output image data (54) of soft biological tissue (12) in particular in real time during surgery, comprising the steps of:
• providing pre-operative three-dimensional image data (6) of the soft biological tissue (12),
• acquiring interoperative image data (14) of the soft biological tissue (12) in at least one of the visible-light spectrum and the near-infrared spectrum, the interoperative image data (14) comprising fluorescent-light image data acquired using fluorescent light from a fluorophore (22),
• computing a blood flow direction (64) at at least one location in the interoperative image data (14), using the fluorescent-light image data,
• automatically identifying at least one vascular plexus structure (48) in the interoperative image data (14), using the interoperative image data (14) and the computed blood flow direction (64),
• identifying the at least one identified vascular plexus structure (48) in the pre-operative three-dimensional image data (6),
• elastic matching of the pre-operative three-dimensional image data (6) to the interoperative image data (14) based on the at least one identified vascular plexus structure (48),
• forming the output image data (54) from the elastically matched pre-operative three-dimensional image data (6) and the interoperative image data (14).

2. Image processing method according to claim 1, wherein the identified vascular plexus structure (48) in the interoperative image data (14) has been recorded using light in the visible spectrum.

3. Image processing method according to any one of claims 1 or 2, wherein the output image data (54) are displayed.

4. Image processing method according to any of claims 1 to 3, wherein at least one optical filter arrangement (26) for reducing specular reflections is used for acquiring the interoperative image data (14).

5. Image processing method according to any one claims 1 to 4, wherein positioning data (60) are acquired, the positioning data (60) being representative of at least one of the focal length of an optical lens system (62) used for acquiring the interoperative image data (14), the field depth of the optical lens system (62), the distance setting of the optical lens system (62), a size, a dimension and an orientation of a field of view (10) of the optical lens system (62), at the time of acquiring the interoperative image data (14), and wherein the positioning data (60) are used in at least one of identifying the vascular plexus structure (48) in the pre-operative three-dimensional image data (6), elastically matching the pre-operative three-dimensional image data (6) to the interoperative image data (14) and displaying the output image data (54).

6. Medical observation device (1) for generation of output image data (54) of soft biological tissue (12), in particular during surgery, such as a microscope (2) or endoscope, the device comprising:
• a memory assembly (4) comprising pre-operative three-dimensional image data (6) of the soft biological tissue (12),
• a camera assembly (8) configured to acquire interoperative image data (14) of the soft biological tissue (12) in at least one of the visible-light spectrum and the near-infrared spectrum the interoperative image data (14) comprising fluorescent-light image data acquired using fluorescent light from a fluorophore (22),
• an image processor assembly (40) that is configured to compute a blood flow direction (64) at at least one location in the interoperative image data (14), using the fluorescent-light image data, the image processor assembly (40) comprising:
o a pattern-matching module (46) configured to identify at least one vascular plexus structure (48) in the interoperative image data (14), using the interoperative image data (14) and the computed blood flow direction (64), and to identify the at least one identified vascular plexus structure (48) in the pre-operative three-dimensional image data (6),
o a matching module (50) configured to elastically match the pre-operative three-dimensional image data (6) to the interoperative image data (14) based on the at least one identified vascular plexus structure (48), and
o an image-forming module (52) configured to combine at least part of the elastically matched pre-operative three-dimensional image data (6) with the interoperative image data (14) to the output image data (54), and
• an output interface configured to output the output image data (54); and wherein
• the image-forming module (52) is adapted to combine the identified vascular plexus structure (48) in the output image data (54) with time-varying marker data which are representative of the blood flow direction (64).

7. Medical observation device (1) according to claim 6, further comprising a display assembly (56) for displaying the output image data (54).

8. Medical observation device (1) according to claim 6 or 7, wherein the camera assembly (8) comprises at least one filter arrangement (26) configured to reduce specular reflections, the filter arrangement (26) comprising at least one pair of cross-polarizers.

9. Medical observation device (1) according to any one of claims 6 to 8, wherein the camera assembly (8) comprises at least one filter arrangement (28) having a pass band matched to the fluorescence spectrum of at least one fluorophore (22).

10. Medical observation device (1) according to any one of claims 6 to 9, wherein the camera assembly (8) comprises an optical lens system (62) and at least one position sensor (58) for acquiring positioning data (60), the positioning data (60) being representative of at least one of the focal length, field depth and distance setting of the optical lens system (62), a size, dimension and orientation of the field of view (10).

11. Non-transitory computer-readable medium storing a programme causing the medical observation device according to any one of claims 6 to 10 to execute the image processing method according to any one of claims 1 to 5.

## Patentansprüche

1. Bildverarbeitungsverfahren zum Anzeigen von Ausgabebilddaten (54) eines weichen biologischen Gewebes (12) insbesondere in Echtzeit während eines chirurgischen Eingriffs, das die folgenden Schritte umfasst:
- Vorsehen von präoperativen dreidimensionalen Bilddaten (6) des weichen biologischen Gewebes (12),
- Erhalten von intraoperativen Bilddaten (14) des weichen biologischen Gewebes (12) im sichtbaren Lichtspektrum und/oder Nahinfrarotspektrum, wobei die intraoperativen Bilddaten (14) Fluoreszenzlicht-Bilddaten, die unter Verwendung eines Fluoreszenzlichts von einem Fluorophor (22) erhalten werden, umfassen,
- Berechnen einer Blutflussrichtung (64) an wenigstens einer Position in den intraoperativen Bilddaten (14) unter Verwendung der Fluoreszenzlicht-Bilddaten,
- automatisches Identifizieren wenigstens einer Gefäßplexusstruktur (48) in den intraoperativen Bilddaten (14) unter Verwendung der intraoperativen Bilddaten (14) und der berechneten Blutflussrichtung (64),
- Identifizieren der wenigstens einen identifizierten Gefäßplexusstruktur (48) in den präoperativen dreidimensionalen Bilddaten (6),
- elastisches Abgleichen der präoperativen dreidimensionalen Bilddaten (6) mit den intraoperativen Bilddaten (14) basierend auf der wenigstens einen identifizierten Gefäßplexusstruktur (48),
- Erstellen der Ausgabebilddaten (54) aus den elastisch abgeglichen präoperativen dreidimensionalen Bilddaten (6) und den intraoperativen Bilddaten (14).

2. Bildverarbeitungsverfahren nach Anspruch 1, wobei die identifizierte Gefäßplexusstruktur (48) in den intraoperativen Bilddaten (14) unter Verwendung von Licht im sichtbaren Spektrum aufgezeichnet wurde.

3. Bildverarbeitungsverfahren nach Anspruch 1 oder 2, wobei die Ausgabebilddaten (54) angezeigt werden.

4. Bildverarbeitungsverfahren nach einem der Ansprüche 1 bis 3, wobei wenigstens eine optische Filteranordnung (26) zum Reduzieren von Spiegelreflexionen für das Erhalten der intraoperativen Bilddaten (14) verwendet wird.

5. Bildverarbeitungsverfahren nach einem der Ansprüche 1 bis 4, wobei Positionierungsdaten (60) erhalten werden, wobei die Positionierungsdaten (60) die Brennweite eines optischen Linsensystems (62), das für das Erhalten der intraoperativen Bilddaten (14) verwendet wird, die Feldtiefe des optischen Linsensystems (62), die Distanzeinstellung des optischen Linsensystems (62) und/oder die Größe, Dimension und Ausrichtung eines Sichtfelds (10) des optischen Linsensystems (62) zum Zeitpunkt des Erhaltens der intraoperativen Bilddaten (14) wiedergeben und wobei die Positionierungsdaten (60) beim Identifizieren der Gefäßplexusstruktur (48) in den präoperativen dreidimensionalen Bilddaten (6), beim elastischen Abgleichen der präoperativen dreidimensionalen Bilddaten (6) mit den intraoperativen Bilddaten (14) und/oder beim Anzeigen der Ausgabebilddaten (54) verwendet werden.

6. Medizinisches Beobachtungsgerät (1) für das Erzeugen von Ausgabebilddaten (54) eines weichen biologischen Gewebes (12) insbesondere während eines chirurgischen Eingriffs wie etwa ein Mikroskop (2) oder Endoskop, wobei das Gerät umfasst:
- eine Speicheranordnung (4), die präoperative dreidimensionale Bilddaten (6) des weichen biologischen Gewebes (12) enthält,
- eine Kameraanordnung (8), die konfiguriert ist zum Erhalten von intraoperativen Bilddaten (14) des weichen biologischen Gewebes (12) im sichtbaren Lichtspektrum und/oder Nahinfrarotspektrum, wobei die intraoperativen Bilddaten (14) Fluoreszenzlicht-Bilddaten, die unter Verwendung eines Fluoreszenzlichts von einem Fluorophor (22) erhalten werden, umfassen,
- eine Bildprozessoranordnung (40), die konfiguriert ist zum Berechnen einer Blutflussrichtung (64) an wenigstens einer Position in den intraoperativen Bilddaten (14) unter Verwendung der Fluoreszenzlicht-Bilddaten, wobei die Bildprozessoranordnung (40) umfasst:
- ein Musterabgleichmodul (46), das konfiguriert ist zum Identifizieren wenigstens einer Gefäßplexusstruktur (48) in den intraoperativen Bilddaten (14) unter Verwendung der intraoperativen Bilddaten (14) und der berechneten Blutflussrichtung (64) und zum Identifizieren der wenigstens einen identifizierten Gefäßplexusstruktur (48) in den präoperativen dreidimensionalen Bilddaten (6),
- ein Abgleichmodul (50), das konfiguriert ist zum elastischen Abgleichen der präoperativen dreidimensionalen Bilddaten (6) mit den intraoperativen Bilddaten (14) basierend auf der wenigstens einen identifizierten Gefäßplexusstruktur (48), und
- ein Bilderstellungsmodul (52), das konfiguriert ist zum Kombinieren wenigstens eines Teils der elastisch mit den intraoperativen Bilddaten (14) abgeglichenen präoperativen dreidimensionalen Bilddaten (6) mit den Ausgabebilddaten (54), und
- eine Ausgabeschnittstelle, die konfiguriert ist zum Ausgeben der Ausgabebilddaten (54),
wobei das Bilderstellungsmodul (52) ausgebildet ist zum Kombinieren der identifizierten Gefäßplexusstruktur (48) in den Ausgabebilddaten (54) mit in der Zeit variierenden Markerdaten, die die Blutflussrichtung (64) wiedergeben.

7. Medizinisches Beobachtungsgerät (1) nach Anspruch 6, das weiterhin eine Displayanordnung (56) für das Anzeigen der Ausgabebilddaten (54) umfasst.

8. Medizinisches Beobachtungsgerät (1) nach Anspruch 6 oder 7, wobei die Kameraanordnung (8) wenigstens eine Filteranordnung (26) umfasst, die konfiguriert ist zum Reduzieren von Spiegelreflexionen, wobei die Filteranordnung (26) wenigstens ein Paar von Kreuzpolarisatoren umfasst.

9. Medizinisches Beobachtungsgerät (1) nach einem der Ansprüche 6 bis 8, wobei die Kameraanordnung (8) wenigstens eine Filteranordnung (28), die ein mit dem Fluoreszenzspektrum des wenigstens einen Fluorophors (22) abgeglichenes Durchlassband aufweist, umfasst.

10. Medizinisches Beobachtungsgerät (1) nach einem der Ansprüche 6 bis 9, wobei die Kameraanordnung (8) ein optisches Linsensystem (62) und wenigstens einen Positionssensor (58) für das Erhalten von Positionierungsdaten (60) umfasst, wobei die Positionierungsdaten (60) die Brennweite, die Feldtiefe und die Distanzeinstellung des optischen Linsensystems (62) und/oder die Größe, Dimension und Ausrichtung des Sichtfelds (10) wiedergeben.

11. Nicht-transitorisches, computerlesbares Medium mit einem darauf gespeicherten Programm, das das medizinische Beobachtungsgerät gemäß einem der Ansprüche 6 bis 10 zum Ausführen des Bildverarbeitungsverfahrens gemäß einem der Ansprüche 1 bis 5 veranlasst.

## Revendications

1. Procédé de traitement d'image pour afficher des données d'image de sortie (54) de tissu biologique mou (12) en particulier en temps réel durant la chirurgie, comprenant les étapes de :
• fourniture de données d'image tridimensionnelles pré-opératoires (6) du tissu biologique mou (12),
• acquisition des données d'image inter-opératoires (14) du tissu biologique mou (12) dans au moins l'un du spectre de la lumière visible et du spectre proche-infrarouge, les données d'image inter-opératoires (14) comprenant des données d'image de lumière fluorescente acquises en utilisant de la lumière fluorescente provenant d'un fluorophore (22),
• calcul d'un sens d'écoulement du sang (64) au niveau d'au moins un emplacement dans les données d'image inter-opératoires (14), en utilisant les données d'image de lumière fluorescente,
• identification automatiquement d'au moins une structure de plexus vasculaire (48) dans les données d'image inter-opératoires (14), en utilisant les données d'image inter-opératoires (14) et le sens calculé d'écoulement du sang (64),
• identification de la au moins une structure identifiée de plexus vasculaire (48) dans les données d'image tridimensionnelles pré-opératoires (6),
• mise en correspondance élastique des données d'image tridimensionnelles pré-opératoires (6) avec les données d'image inter-opératoires (14) sur la base de la au moins une structure identifiée de plexus vasculaire (48),
• mise en forme des données d'image de sortie (54) à partir des données d'image tridimensionnelles préopératoires (6) élastiquement en correspondance et des données d'image inter-opératoires (14).

2. Procédé de traitement d'image selon la revendication 1, la structure identifiée de plexus vasculaire (48) dans les données d'image inter-opératoires (14) ayant été enregistrée en utilisant la lumière dans le spectre visible.

3. Procédé de traitement d'image selon l'une quelconque des revendications 1 ou 2, les données d'image de sortie (54) étant affichées.

4. Procédé de traitement d'image selon l'une quelconque des revendications 1 à 3, au moins une configuration de filtre optique (26) pour réduire les reflets spéculaires étant utilisée pour acquérir les données d'image inter-opératoires (14).

5. Procédé de traitement d'image selon l'une quelconque des revendications 1 à 4, les données de positionnement (60) étant acquises, les données de positionnement (60) étant représentatives d'au moins l'un de la distance focale d'un système de lentilles optiques (62) utilisé pour acquérir les données d'image inter-opératoires (14), de la profondeur de champ du système de lentilles optiques (62), du réglage de distance du système de lentilles optiques (62), d'une taille, d'une dimension et d'une orientation d'un champ de vision (10) du système de lentilles optiques (62), au moment de l'acquisition des données d'image inter-opératoires (14), et les données de positionnement (60) étant utilisées dans au moins l'une de l'identification de la structure de plexus vasculaire (48) dans les données d'image tridimensionnelles pré-opératoires (6), de la mise en correspondance élastiquement des données d'image tridimensionnelles pré-opératoires (6) avec les données d'image inter-opératoires (14) et de l'affichage des données d'image de sortie (54).

6. Dispositif d'observation médicale (1) pour la génération de données d'image de sortie (54) de tissu biologique mou (12), en particulier durant la chirurgie, tel qu'un microscope (2) ou un endoscope, le dispositif comprenant :
• un ensemble mémoire (4) comprenant des données d'image tridimensionnelles pré-opératoires (6) du tissu biologique mou (12),
• un ensemble caméra (8) conçu pour acquérir des données d'image inter-opératoires (14) du tissu biologique mou (12) dans au moins l'un du spectre de la lumière visible et du spectre proche-infrarouge, les données d'image inter-opératoires (14) comprenant des données d'image de lumière fluorescente acquises en utilisant la lumière fluorescente provenant d'un fluorophore (22),
• un ensemble de traitement d'image (40) qui est conçu pour calculer un sens d'écoulement du sang (64) au niveau d'au moins un emplacement dans les données d'image inter-opératoires (14), en utilisant les données d'image de lumière fluorescente, l'ensemble de traitement d'image (40) comprenant :
o un module de mise en correspondance de motifs (46) conçu pour identifier au moins une structure de plexus vasculaire (48) dans les données d'image inter-opératoires (14), en utilisant les données d'image inter-opératoires (14) et le sens calculé d'écoulement du sang (64), et pour identifier la au moins une structure identifiée de plexus vasculaire (48) dans les données d'image tridimensionnelles pré-opératoires (6),
o un module de mise en correspondance (50) conçu pour faire correspondre élastiquement les données d'image tridimensionnelles pré-opératoires (6) avec les données d'image inter-opératoires (14) sur la base de la au moins une structure identifiée de plexus vasculaire (48), et
o un module de mise en forme d'image (52) conçu pour combiner au moins une partie des données d'image tridimensionnelles préopératoires (6) élastiquement mises en correspondance aux données d'image inter-opératoires (14) vers les données d'image de sortie (54), et
• une interface de sortie conçue pour émettre les données d'image de sortie (54) ; et
• le module de mise en forme d'image (52) étant adapté pour combiner la structure identifiée de plexus vasculaire (48) dans les données d'image de sortie (54) à des données marqueurs qui varient dans le temps et qui sont représentatives du sens d'écoulement du sang (64).

7. Dispositif d'observation médicale (1) selon la revendication 6, comprenant en outre un ensemble d'affichage (56) pour afficher les données d'image de sortie (54).

8. Dispositif d'observation médicale (1) selon la revendication 6 ou 7, l'ensemble caméra (8) comprenant au moins une configuration de filtre (26) conçue pour réduire les reflets spéculaires, la configuration de filtre (26) comprenant au moins une paire de polariseurs croisés.

9. Dispositif d'observation médicale (1) selon l'une quelconque des revendications 6 à 8, l'ensemble caméra (8) comprenant au moins une configuration de filtre (28) ayant une bande passante qui correspond au spectre de fluorescence d'au moins un fluorophore (22).

10. Dispositif d'observation médicale (1) selon l'une quelconque des revendications 6 à 9, l'ensemble caméra (8) comprenant un système de lentilles optiques (62) et au moins un capteur de position (58) pour acquérir des données de positionnement (60), les données de positionnement (60) étant représentatives d'au moins l'un de la distance focale, de la profondeur de champ et du réglage de distance du système de lentilles optiques (62), d'une taille, d'une dimension et de l'orientation du champ de vision (10).

11. Support non temporaire pouvant être lu par ordinateur stockant un programme amenant le dispositif d'observation médicale selon l'une quelconque des revendications 6 à 10 à exécuter le procédé de traitement d'image selon l'une quelconque des revendications 1 à 5.
